(19) Europäisches Patentamt
European Patent Office
Office européen des brevets

(11) Veröffentlichungsnummer: **0 351 645 B1**

(12) **EUROPÄISCHE PATENTSCHRIFT**

(45) Veröffentlichungstag der Patentschrift: **24.02.93**

(51) Int. Cl.$^5$: **A61K 7/13**

(21) Anmeldenummer: **89112273.1**

(22) Anmeldetag: **05.07.89**

Verbunden mit 89907730.9/0428538
(europäische
Anmeldenummer/Veröffentlichungsnummer)
durch Entscheidung vom 15.10.91.

# (54) Haarfärbemittel-Zubereitung.

(30) Priorität: **14.07.88 DE 3823843**

(43) Veröffentlichungstag der Anmeldung:
**24.01.90 Patentblatt 90/04**

(45) Bekanntmachung des Hinweises auf die Patenterteilung:
**24.02.93 Patentblatt 93/08**

(84) Benannte Vertragsstaaten:
**AT BE CH DE ES FR GB GR IT LI NL SE**

(56) Entgegenhaltungen:
**EP-A- 0 188 216**
**EP-A- 0 190 708**

(73) Patentinhaber: **Henkel Kommanditgesellschaft auf Aktien**
**Postfach 1100 Henkelstrasse 67**
**W-4000 Düsseldorf 1(DE)**

(72) Erfinder: **Höffkes, Horst, Dr.**
**Carlo-Schmid-Strasse 114**
**W-4000 Düsseldorf-Hellerhof(DE)**
Erfinder: **Lange, Fritz, Dr.**
**Bühne 22**
**W-4300 Essen(DE)**
Erfinder: **Schrader, Dieter**
**Itterstrasse 7**
**W-4000 Düseldorf(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

**Beschreibung**

Gegenstand der Erfindung ist eine flüssige Zubereitung für Oxidationshaarfärbemittel. Derartige Zubereitungen bestehen aus Haarfarbstoffvorprodukten und einem für die Anwendung auf dem Haar geeigneten Träger. Bevorzugte Träger sind cremeförmige Emulsionen vom Typ Wasser-in-Öl und wäßrige oder wäßrigalkoholische Lösungen von Seifen.

Derartige Oxidationshaarfärbemittel-Grundlagen auf Basis flüssiger, wäßriger oder wäßrig-alkoholischer Seifenlösungen bilden nach Zugabe der zur Entwicklung der Oxidationsfarben erforderlichen wäßrigen Lösung des Oxidationsmittels ein dickflüssiges bis gelförmiges gebrauchsfertiges Haarfärbepräparat aus.

Es ist weiterhin bekannt, Haarfärbemittel-Zubereitungen mit einem Zusatz an wasserlöslichen kationischen Polymeren zu versehen. Dadurch werden die haarkosmetischen Eigenschaften des behandelten Haares verbessert; gleichzeitig wird eine haaravivierende Wirkung erzielt.

Ein Problem bereitet jedoch die Herstellung flüssiger Zubereitungen für Oxidationshaarfärbemittel mit einem Gehalt an Seifen und kationischen Polymeren, da solche Zubereitungen zu Inhomogenität und in manchen Fällen bereits unmittelbar nach der Herstellung oder nach längerer Lagerung zur Bildung von Trübungen, Ausfällungen und Bodensatz neigen. Des weiteren geht die haaravivierende Wirkung des Produktes durch Interaktion des kationischen Polymeren mit der Seife verloren.

Oxidationshaarfärbemittel-Zubereitungen auf Basis flüssiger, wäßriger oder wäßrig-alkoholischer Seifenlösungen stellen andererseits aber eine vorteilhafte Konfektionierungsform dar, weil sie nach Zugabe der wäßrigen Lösung des Oxidationsmittels eine dickflüssige bis gelförmige Färbezubereitung ausbilden, die bei der Anwendung am Haar haftet.

Um Seifen und kationische Polymere in Haarfärbemittel-Grundlagen einarbeiten zu können, wurde daher in der DE-OS 35 00 877 vorgeschlagen, zu Stabilisierung der Formulierungen entweder Dicarbonsäuren in Form ihrer wasserlöslichen Salze oder Amine der allgemeinen Formel (D)

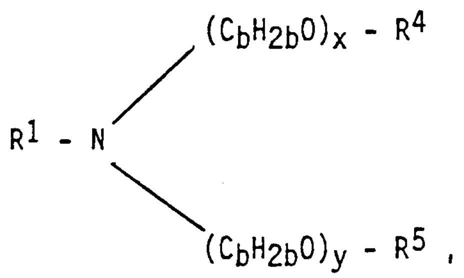

(D)

einzusetzen, in der $R^1$ eine Alkylgruppe mit 8 - 22 C-Atomen darstellt und $R^4$ und $R^5$ unabhängig voneinander Wasserstoff oder eine Acylgruppe der Formel $R^6$-COO- sind, wobei $R^6$ eine Alkyl- oder Alkenylgruppen mit 1-21 C-Atomen ist, b = 2 oder 3 und x und y gleich 0 - 5 sind, wobei die Summe (x + y) 2 bis 6 betragen kann.

Wenngleich die wäßrigen Systeme mit Seife und kationischem Polymer durch die genannten Substanzen unter Erhaltung der avivierenden Eigenschaften der kationischen Polymeren stabilisiert werden, verbleibt jedoch der Nachteil, daß relativ hohe Konzentrationen, insbesondere des Amins, eingesetzt werden müssen, um die zur Stabilisierung notwendigen hohen Viskositäten zu erzielen.

Es bestand daher die Aufgabe, stabilisierende Substanzen zu finden, deren Verwendung bereits in niedrigeren Konzentrationen zu einem hohen Viskositätsanstieg der Formulierung führt.

Es wurde nun überraschenderweise gefunden, daß bestimmte Amine mit einer von den Aminen der Formel (D) abweichenden Struktur die gewünschten Eigenschaften aufweisen.

Gegenstand der Erfindung ist daher eine flüssige Zubereitung für Oxidationshaarfärbemittel, bestehend aus Haarfarbstoff-Vorprodukten und einem wäßrigen oder wäßrig-alkoholischen Träger, enthaltend Seifen (A) und wasserlösliche kationische Polymere (B), dadurch gekennzeichnet, daß sie zur Stabilisierung Amine der Struktur (C)

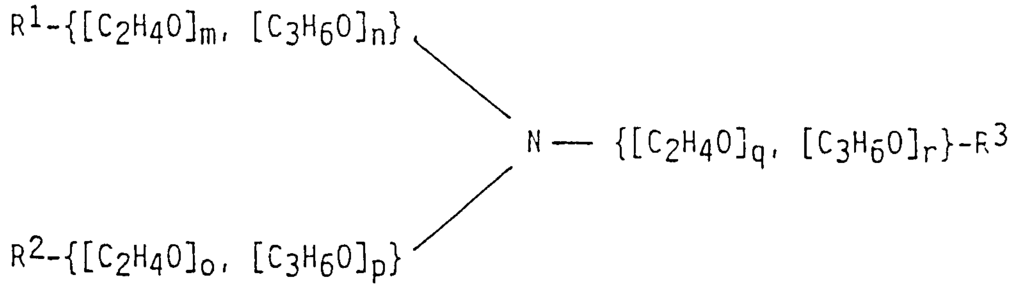 (C),

in denen

| | |
|---|---|
| m, n, o, p, q und r | 0 oder Zahlen bis 6 sind, |
| $R^1$ | eine Alkyl-Gruppe mit 8 bis 22 C-Atomen, |
| $R^2$ | Wasserstoff oder eine Alkyl-Gruppe mit 8 bis 22 C-Atomen, |
| $R^3$ | Wasserstoff und, falls r = q = 0 ist, Alkyl mit 1 bis 4 Kohlenstoffatomen darstellt und im Falle m = n = 0 $R^2$ und $R^3$ nicht gleichzeitig für Wasserstoff stehen, |

enthält.

Weiterhin wurde überraschenderweise gefunden, daß ein synergistischer Effekt hinsichtlich des Viskositätsaufbaus auftritt, wenn die genannten Amine in Kombination mit Aminen der allgemeinen Struktur (D)

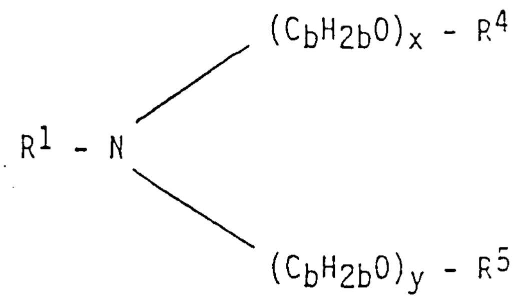 (D)

eingesetzt werden, in der $R^4$ und $R^5$ unabhängig voneinander Wasserstoff oder eine Acylgruppe der Formel $R^6$-COO- bedeutet, wobei $R^6$ eine Alkyl- oder Alkenylgruppe mit 1-21 C-Atomen ist, b = 2 oder 3 und x und y gleich 0 - 5 sind, wobei die Summe (x + y) 2 bis 6 betragen kann.

Es sind daher solche flüssigen Zubereitungen für Oxidationsfärbemittel bevorzugt, die zur Stabilisierung eine Kombination von Aminen (C) mit Aminen (D) enthalten.

Amine der Struktur (C) sind bereits aus der DE-OS 35 04 242 als grenzflächenaktive Stoffe bekannt. Hinweise auf eine stabilisierende Wirkung für Lösungen, die Seifen neben kationischen Polymeren enthalten, sind der Druckschrift aber nicht zu entnehmen. Die Amine der Struktur (C) sind nach der Lehre der genannten Druckschrift auf einfache Weise durch Umsetzung von beispielsweise Hydroxyethyl-dialkylaminen, Hydroxypropyl-dialkylaminen, Dihydroxyalkylalkylaminen oder Trihydroxyalkylaminen mit Schwefelsäurehalbestern der Formel $R^7$-$\{[OC_2H_4]_t,[OC_3H_6]_z\}$-$OSO_3$-H, wobei $R^7$ steht für eine Alkylgruppe mit 8 - 22 C-Atomen und t und z unabhängig voneinander Zahlen von 0 bis 6 sind, mit der Einschränkung, daß t und z nicht gleichzeitig 0 sind, oder deren Alkali- oder Erdalkalimetallsalzen in Gegenwart von starken Basen zugänglich. Bezüglich der Einzelheiten dieses Herstellungsverfahrens wird ausdrücklich auf die Lehre der Druckschrift verwiesen.

Desweiteren ist es möglich, Amine mit endständigen Hydroxylgruppen nach den bekannten Verfahren bei erhöhter Temperatur und erhöhtem Druck unter Verwendung üblicher Katalysatoren wie Natriummethylat direkt mit Ethylenoxid und/oder Propylenoxid umzusetzen. Die so erhaltenen Amine weisen wiederum endständige Hydroxylgruppen auf, die gewünschtenfalls weiter umgesetzt werden können, beispielsweise mit Schwefelsäurehalbestern nach dem oben beschriebenen Verfahren.

Es ist dem Fachmann bekannt, daß bei Alkoxylierungsreaktionen wie beispielsweise der Anlagerung von n mol Ethylenoxid an 1 mol einer Verbindung mit einem aktiven Wasserstoffatom nach den bekannten Ethoxylierungsverfahren kein einheitliches Addukt, sondern ein Gemisch aus Restmengen freier Ausgangsverbindung und einer Reihe homologer (oligomerer) Anlagerungsprodukte von 1, 2, 3, ... n, n+1, n+2 ...usw. Molekülen Ethylenoxid je Molekül Ausgangsverbindung erhalten wird. Der mittlere Ethoxylierungsgrad (n) wird dabei definiert durch die Ausgangsmengen an Verbindung mit aktivem Wasserstoffatom und Ethylenoxid. Die Verteilungskurve des Homologengemisches weist in der Regel ein Maximum im Bereich zwischen n-3 und n+3 auf. Nähere Informationen zu diesen Punkten können beispielsweise der Zeitschrift Soap/Cosmetics/Chemical Specialities, Heft Januar 1988, S. 34, entnommen werden.

Soweit die in dieser Schrift aufgeführten Produkte daher durch Anlagerung von Ethylenoxid und/oder Propylenoxid an entsprechende Ausgangsverbindungen hergestellt und ohne weitere Fraktionierung des Produktgemisches eingesetzt wurden, handelt es sich bei den angegebenen Alkoxylierungsgraden jeweils um die mittleren Alkoxylierungsgrade des vorliegenden Produktgemisches.

Besonders gute stabilisierende Wirkungen zeigen solche Amine, bei denen $R^1$ eine Alkylgruppe mit 16 bis 18 C-Atomen, $R^2$ Wasserstoff oder eine Alkylgruppe mit 16 bis 18 C-Atomen und $R^3$ Wasserstoff oder, falls r = q = 0 ist, Methyl darstellt, sowie m, o, r Zahlen zwischen 0 und 3 und n, p, q Zahlen zwischen 0 und 1,5 sind, wobei m und n nicht gleichzeitig 0 sind. Üblicherweise zeigen solche Amine die besseren stabilisierenden Wirkungen, deren Ethoxylierungs- und/oder Propoxylierungsgrade im unteren Bereich der für die Zahlen m bis r angegebenen Werte liegen.

Hinsichtlich der terminalen Alkylketten kann es sich bei den Aminen sowohl um einheitliche Produkte, als auch um Mischungen handeln. Letztere werden in der Regel dann vorliegen, wenn als Quelle für diese Alkylgruppen natürliche, nachwachsende Rohstoffe wie Öle und Fette eingesetzt werden. So zeigen Amine, die als Alkylgruppen eine Mischung von Cetyl- und Stearylgruppen im Verhältnis 1:4 bis 2:1 aufweisen, wie sie, beispielsweise aus pflanzlichem oder tierischem Talg gewonnen werden können, sehr gute stabilisierende Eigenschaften. Im Rahmen der erfinderischen Lehre besonders bevorzugte Amine sind daher beispielsweise

(Cetyl/stearyl-oxyethyl)-dihydroxyethyl-amin (C1),
Bis(cetyl/stearyl-oxyethyl)-hydroxyethyl-amin (C2),

desweiteren Verbindungen der Formeln

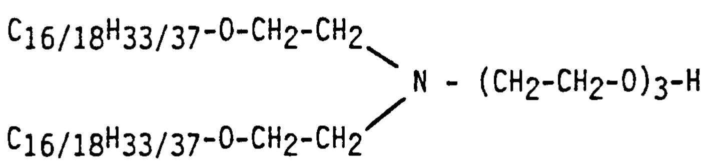

(C3),

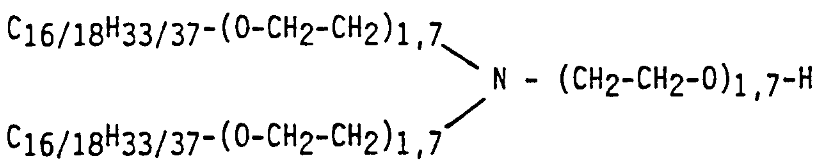

(C4) und

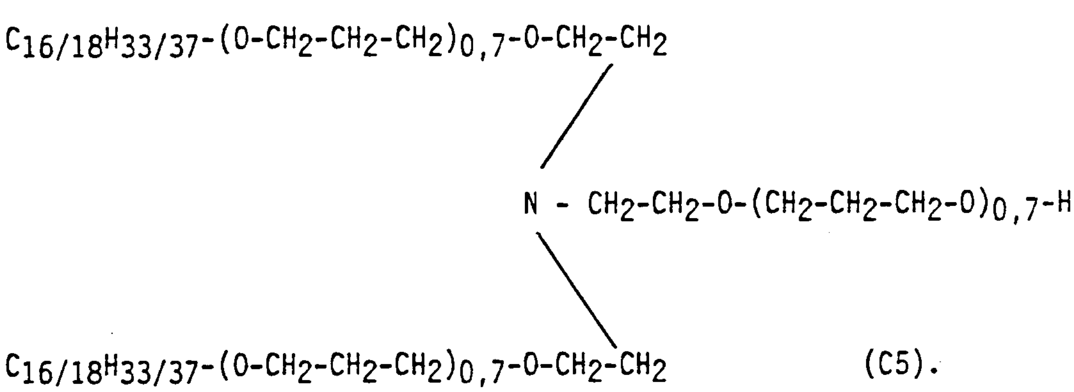

(C5).

$C_{16/18}H_{33/37}$ steht in den vorgenannten Formeln für Gemische von Alkylgruppen, die ganz überwiegend aus Cetyl($C_{16}H_{33}$)- und Stearyl($C_{18}H_{37}$)-Gruppen bestehen.

Amine der oben aufgeführten Formel (D), in der $R^1$, $R^4$, $R^5$, b, x und y die genannten Bedeutungen haben, sind aus primären Fettaminen mit 8-22 C-Atomen durch Anlagerung von (x + y) Mol Ethylenoxid oder Propylenoxid, z.B. nach DE-PS 552 268, zugänglich.
Dafür können sowohl reine Fettamine als auch Mischungen von Fettaminen eingesetzt werden. Es ist bevorzugt, Amin-Mischungen einzusetzen, die aus natürlichen Fetten und Ölen nach den bekannten Verfahren erhalten werden. Als Beispiel sei auf das Kokosalkylamin verwiesen. Durch Anlagerung von Ethylenoxid oder Propylenoxid an die genannten Fettamine werden zunächst alkoxylierte Fettamine der allgemeinen Formel (D) erhalten, in der $R^4$ und $R^5$ Wasserstoff bedeuten. Diese Produkte lassen sich durch Veresterung mit Carbonsäuren der allgemeinen Formel $R^6$-COOH, in der $R^6$ eine Alkylgruppe mit 1-21 C-Atomen ist, oder Methylestern oder Säurechloriden dieser Carbonsäuren in die Produkte der allgemeinen Formel (D) überführen, bei welchen $R^4$ und $R^5$ eine Acylgruppe der Formel $R^6$-COO- darstellt. Zahlreiche Produkte der allgemeinen Formel (D) sind im Handel erhältlich. Ein Anlagerungsprodukt von 3 Mol Ethylenoxid an einen $C_{12}$-$C_{14}$-Fettalkohol wird zum Beispiel unter der Bezeichnung Lowenol$^{(R)}$C-243 verkauft. Ein Bis-(2-hydroxyethyl)-sojaalkylamindioleat ist unter der Bezeichnung Lowenol$^{(R)}$ S-216 erhältlich. Weitere Fettamin-Oxalkylate sind unter den Bezeichnungen Araphen$^{(R)}$, Genamin$^{(R)}$, Marlazin$^{(R)}$ und Lutensol$^{(R)}$ im Handel.

Als Seifen (A) werden bevorzugt wasserlösliche Seifen von Fettsäuren eingesetzt. Hierfür besonders geeignete Fettsäuren sind solche, die bei 20 °C flüssig sind. Dies sind beispielsweise ungesättigte lineare Fettsäuren wie Ölsäure, Linolsäure, Palmitoleinsäure, Erucasäure oder flüssige Gemische dieser Fettsäuren untereinander und mit geringen Anteilen gesättigter linearer Fettsäuren mit 12-22 C-Atomen. Andere bevorzugt geeignete flüssige Fettsäuren sind verzweigte Fettsäuren, z.B. die 2-Hexyl-decansäure, die 2-Octyldodecansäure oder die Isostearinsäure.

Zur Überführung der Fettsäuren in wasserlösliche Seifen eignen sich Alkalihydroxide und Alkalicarbonate, Ammoniak sowie Mono-, Di- und Trialkanolamine mit 2 bis 4 C-Atomen in der Alkanolgruppe. Bevorzugt geeignet ist Ölsäure in Form der Ammonium-, Mono-, Di-und Triethanolammoniumseife.

Als wasserlösliche kationische Polymere (B) kommen prinzipiell alle Polymeren im Molekulargewichtsbereich von 1 000 bis 3 000 000 in Frage, die entweder in der Polymerkette freie oder alkylsubstituierte Aminogruppen oder quartäre Ammoniumgruppen enthalten oder an die Polymerkette direkt oder über Zwischenglieder gebundene primäre, sekundäre oder tertiäre Aminogruppen oder quartäre Ammoniumgruppen tragen. Diese Aminogruppen oder quartären Ammoniumgruppen können auch Glieder von 5- oder 6-gliedrigen Ringsystemen, zum Beispiel dem Morpholin-, Piperidin-, Piperazin-oder Imidazol-Ringsystem sein. Zahlreiche Beispiele für solche wasserlöslichen kationischen Polymeren sind zum Beispiel in der DE-OS 28 11 010 näher beschrieben. Darüber hinaus sind zahlreiche weitere wasserlösliche kationische Polymere literaturbekannt.

Bevorzugt geeignet sind wasserlösliche Homo- oder Mischpolymerisate (B1) mit Einheiten der allgemeinen Formel

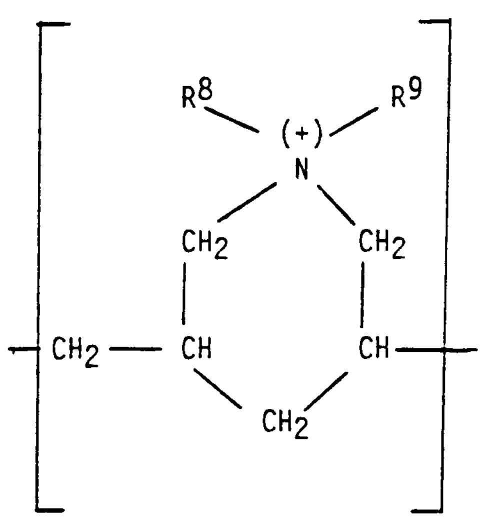

$X^{(-)}$

in der $R^8$ und $R^9$ Alkylgruppen mit 1 bis 4 C-Atomen oder Hydroxyalkylgruppen mit 2 bis 4 C-Atomen sind und $X^{(-)}$ ein Chlorid, Bromid, Hydrogensulfat, Methoxysulfat-, Phosphat- oder Acetat-Anion ist. Beispiele für kationische Polymere dieser Art sind zum Beispiel die Handelsprodukte Merquat$^{(R)}$ 100 und Merquat$^{(R)}$ 550 (Quaternium 41). Die Herstellung dieser Polymeren ist zum Beispiel aus der DE-OS 21 09 081 bekannt.

Weitere bevorzugt geeignete kationische Polymere sind Celluloseether (B2), deren Anhydroglucoseeinheiten 1 bis 3 über Ethersauerstoff gebundene Substituenten mit quartären Ammoniumgruppen tragen. Solche Polymeren sind zum Beispiel aus DE-OS 15 93 657 bekannt. Ein Handesprodukt dieser Struktur ist zum Beispiel Polymer JR$^{(R)}$ 400.

Bevorzugt geeignet sind auch die quartären polymeren Harnstoffderivate (B3), wie sie aus der US-PS 4,157,388 bekannt sind. Ein Handelsprodukt dieses Typs ist Mirapol$^{(R)}$ A15, welches aus Struktureinheiten der allgemeinen Formel

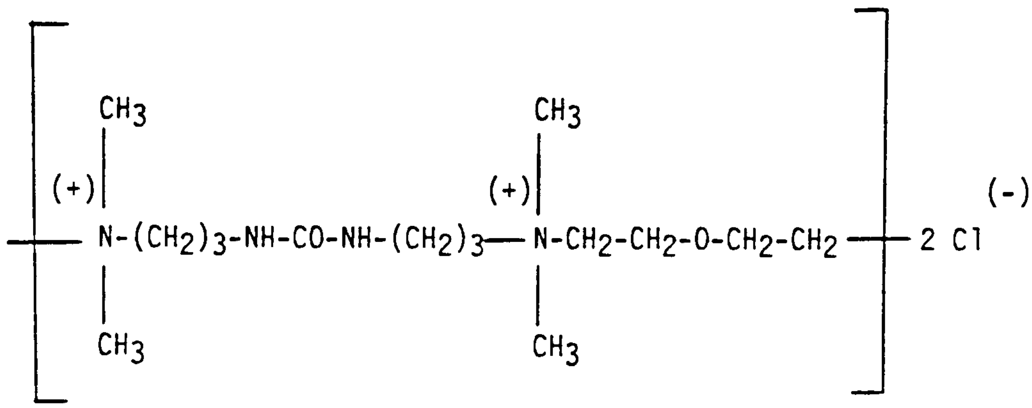

besteht, wobei der mittlere Polymerisationsgrad etwa 6 ist.

Im Rahmen der erfindungsgemäßen Lehre sind solche flüssigen Zubereitungen bevorzugt, die 1-30 Gew.-% Seifen (A), 0,5-10 Gew.-% wasserlösliche kationische Polymere (B), 0,1-20 Gew.-%, insbesondere 0,5-10 Gew.-%, Amine (C) und 0-20 Gew.-%, insbesondere 0-5 Gew.-%, Amine (D) enthalten.

Die erfindungsgemäßen Haarfärbemittel-Zubereitungen enthalten neben den genannten Trägerkomponenten Oxidationshaarfarbstoff-Vorprodukte. Als solche werden die bekannten Farbbasen bzw. Entwicklerverbindungen und bekannte Modifikatoren bzw. Kupplerverbindungen eingesetzt. Die Oxidationsfarbstoffe bilden sich durch oxidative Kupplung einer oder mehrerer Entwicklerkomponenten untereinander oder mit einer oder mehreren Kupplerkomponenten in Gegenwart eines Oxidationsmittels aus. Als Entwicklersubstanzen werden üblicherweise primäre aromatische Amine mit einer weiteren in Para- oder Orthoposition befindlichen freien oder substituierten Hydroxy- oder Aminogruppe, Diaminopyridin-Derivate, heterocyclische Hydrazonderivate, 4-Aminopyrazolonderivate oder Tetraminopyrimidine eingesetzt. Als sogenannte Kupplersubstanzen werden zum Beispiel m-Phenylendiaminderivate, Phenole, Naphthole, Resorcinderivate oder Pyrazolone verwendet. Die erfindungsgemäßen Haarfärbemittel-Zubereitungen können solche Oxidationshaarfarbstoff-Vorproduktein einer Menge von 0,05 bis 5,0 Gew.-%, bevorzugt von 0,2 bis 2,0 Gew.-%, enthalten.

Als weitere Hilfsmittel können die erfindungsgemäßen Haarfärbemittel-Zubereitungen noch synthetische anionische, nicht-ionogene, ampholytische oder zwitterionische Waschrohstoffe in Mengen bis zu 20 Gew.-% enthalten. Geeignet sind zum Beispiel lineare Alkylsulfate mit 12 - 18 C-Atomen in der Alkylgruppe, Alkylpolyglykolethersulfate mit 12 - 16 C-Atomen in der Alkylgruppe und 1 bis 6 Glykolethergruppen im Molekül, Fettalkoholpolyglykolether, die durch Anlagerung von 6 bis 20 Mol Ethylenoxid an $C_{10}$-$C_{18}$-Fettalkohole erhalten werden, Anlagerungsprodukte von 6 bis 20 Mol Ethylenoxid an Nonyl- oder Dodecylphenol, Fettalkyl-dimethylaminoxide, Fettsäuremono- oder diethanolamide, N-Fettalkyl-dimethylglycin, N-Fettalkylaminopropionsäure und andere bekannte oberflächenaktive Stoffe.

Außerdem können die erfindungsgemäßen Haarfärbemittel-Zubereitungen 0 bis 20 Gew.-% Fettalkohole mit 12 bis 22 C-Atomen, zum Beispiel Kokosfettalkohol, Laurylalkohol, Myristylalkohol, Cetylalkohol, Oleylalkohol oder Stearylalkohol in emulgierter Form enthalten. Geeignet sind auch synthetische, verzweigte Alkohole, zum Beispiel 2-Octyldodecanol, 2-Hexyl-decanol, Isostearylalkohol, Isohexadecylalkohol.

Bevorzugt enthalten die erfindungsgemäßen Haarfärbemittel-Zubereitungen niedere Alkohole mit 1 bis 4 C-Atomen und/oder niedere Glykole mit 2 bis 6 C-Atomen, zum Beispiel Ethanol, Isopropanol, n-Propanol, Ethylenglykol, 1,2-Propylenglykol, Methylglykol, Ethylglykol, Butylglykol, Diethylenglykol, Dipropylenglykol oder Hexylenglykol. Diese niederen Alkohole oder Glykole sind bevorzugt in einer Menge von insgesamt 1 bis 30 Gew.-% in den Zubereitungen enthalten. Durch den Zusatz dieser niederen Alkohole und/oder

Polyole bleiben die Zubereitungen bei 20 °C dünnflüssig und leicht verarbeitbar.

Erst bei Zugabe einer etwa gleichen Menge Wasser oder wäßriger Wasserstoffperoxidlösung, wie dies zur Entwicklung der Färbung kurz vor der Anwendung auf dem Haar erfolgt, bildet sich dann ein dickflüssiges bis gelförmiges gebrauchsfertiges Haarfärbepräparat.

Neben den bisher genannten Komponenten enthalten die erfindungsgemäßen Haarfärbemittel-Zubereitungen noch die in solchen Oxidationsfarbstoff-Grundlagenüblichen Zusätze zur Stabilisierung der Oxidationsfarbstoff-Vorprodukte; dies sind Komplexbildner, z. B. Ethylendiaminotetraessigsäure, Nitrilotriessigsäure, 1-Hydroxyethan-1,1-diphosphonsäure oder andere Organodiphosphonsäuren in Form ihrer Alkalisalze, Antioxidantien, wie z. B. Natriumsulfit, Natriumbisulfit, Hydrochinon oder Salze der Thioglycolsäure oder Ascorbinsäure, Puffersalze, wie z. B. Ammoniumsulfat, Ammoniumcarbonat, Ammoniumcitrat sowie Ammoniak oder ein Alkanolamin zur Einstellung eines pH-Wertes von 8 - 10.

Die folgenden Beispiele sollen den Gegenstand der Erfindung näher erläutern.

Beispiele

## I. Herstellung der Amine (C)

a) Amin C1

Zu 10,7 kg (72 mol) Triethanolamin wurden bei Temperaturen von etwa 80 - 100 °C unter Wasserstrahl-Vakuum portionsweise 21 kg Sulfopon(R)T55 (Talgfettalkoholsulfat-Natriumsalz, im wesentlichen Alkylketten der Länge $C_{16}$ und $C_{18}$ im Verhältnis 30 : 70, ca. 55 % Aktivsubstanz) gegeben und dabei entwässert. Nach Belüften mit Stickstoffgas wurden 1,46 kg (36,5 mol) NaOH zugegeben. Anschließend wurde bei 170 °C 5 Stunden erhitzt und dann auf 90 °C abgekühlt.

Nach dem Waschen des Produktes mit heißem Wasser und Abziehen von Verunreinigungen und Nebenprodukten durch Anlegen von Vakuum bei ca. 110 °C wurden 12,5 kg Amin C1 (99,7 % d. Th.) erhalten. Die Aminzahl des Produktes betrug 135,1 (ber. 136,0).

b) Amin C2

Zu 8,0 kg Amin C1 wurden bei ca. 85 °C 13,4 kg Sulfopon(R)T55 (Talgfettalkoholsulfat-Natriumsalz, im wesentlichen Alkylketten der Länge $C_{16}$ und $C_{18}$ im Verhältnis 30 : 70, ca. 55 % Aktivsubstanz) gegeben. Sodann wurde bei ca. 100 °C und angelegtem Vakuum das im Gemisch befindliche Wasser abdestilliert. Nach Zugabe von 0,8 kg NaOH betrug die Reaktionszeit 3 Stunden bei 170 °C und unter Stickstoffatmosphäre. Nach dem Waschen des Produktes mit heißem Wasser und Abziehen von Verunreinigungen und Nebenprodukten durch Anlegen von Vakuum bei ca. 110 °C betrug die Ausbeute an Amin C2 94,2 % d. Th. Die Aminzahl des Produktes war 84 (ber. 83,4).

c) Amin C3

c1) 10,4 kg (70 mol) Triethanolamin, 20 kg (58,3 mol) Lanette(R)E(Natrium-Cetyl-stearyl(1:1)-sulfat, min. 90 % Aktivsubstanz) und 2,8 kg NaOH wurden analog Vorschrift a) umgesetzt. 16,2 kg des Produktes wurden analog Vorschrift b) mit 14,9 kg Lanette E und 2,09 kg NaOH umgesetzt. Die Aminzahl des nunmehr erhaltenen Produktes betrug 84 (ber. 94).

c2) 676 g (1 mol) des so erhaltenen Produktes wurden in einem Autoklaven mit 5,1 g Natriummethylat-Lösung (0,2 Gew.-% Natriummethylat, bezogen auf die Gesamtmenge der Ausgangsstoffe Amin C2 und Ethylenoxid) versetzt. Anschließend wurde mit Stickstoff gespült und 30 Minuten bei 100 °C evakuiert. Sodann wurden bei einer Temperatur von 150 °C 88 g (2 mol) Ethylenoxid unter einem Druck von 5 bar aufgedrückt. Nach einer Nachreaktionszeit von 30 Minuten wurde der Rest des Ethylenoxids bei 75 °C mittels Vakuum abgezogen. Die Ausbeute an Amin C3 betrug 755 g (99 % d. Th.). Die Aminzahl des Produktes betrug 73 (ber. 82).

d) Amin C4

746 g Triethanolamin wurden analog Vorschrift c2) im Autoklaven mit 440 g Ethylenoxid in Anwesenheit von 2,9 g Natriummethylat-Lösung als Katalysator bei 150 °C und einem Druck von 5 bar umgesetzt. Zu 500 g (2,11 mol) des Produktes wurden bei 150-175 °C portionsweise insgesamt 1446 g (4,22 mol) Lanette-(R)E und 169 g (4,22 mol) NaOH in jeweils äquivalenten Mengen hinzugegeben. Das gebildete Produkt

diente im weiteren als Lösungsmittel, so daß die Reaktionsmischung rührbar blieb. Nach Zugabe der letzten Portion Lanette(R)Eund NaOH betrug die weitere Reaktionszeit bei 175 °C 3 Stunden. Anschließend wurde analog Vorschrift a) aufgearbeitet. Als Produkt wurde das Amin C4 in einer Ausbeute von 85 % d. Th. erhalten. Die Aminzahl des Produktes betrug 83,1 (ber. 82,1).

e) Amin C5

Triethanolamin wurde analog Vorschrift c2) mit der doppelten molaren Menge Propylenoxid umgesetzt. 530,4 g (2 mol) des so erhaltenen Produktes wurden mit 4 Portionen aus jeweils 343 g (1 mol) Lanette(R)E und 40 g (1 mol) NaOH umgesetzt. Nach dem Abziehen des Wassers im Vakuum betrugt die Reaktionszeit 3 Stunden bei 150 °C. Anschließend wurde analog Vorschrift a) aufgearbeitet. Als Produkt wurden 1205 g (85 % d. Th.) des Amins C5 erhalten. Die Aminzahl des Produktes betrug 76,5 (ber. 78,8).

## II. Anwendungstechnische Prüfung

Es wurde Zubereitungen folgender Zusammensetzung untersucht:

| Komponente | Gewichtsteile |
|---|---|
| Ölsäure | 1,5 |
| Texapon(R)N 25[1] | 4,0 |
| Dehyton(R)K[2] | 3,0 |
| Polymer JR(R)400[3] | 1,0 |
| Amin (C)[4] | siehe Tabelle 1 |
| Amin (D)[5] | siehe Tabelle 1 |
| Propylenglykol | 2,15 |
| Farbstoff-Vorprodukte | |
| Resorcin | 0,24 |
| 4-Chlorresorcin | 0,11 |
| p-Aminophenolhydrochlorid | 0,06 |
| 2,4-Dichlor-3-aminophenolhydrochlorid | 0,035 |
| p-Phenylendiamin | 0,26 |
| Stabilisator-Komponenten | |
| Ammoniumsulfat | 0,75 |
| Turpinal(R)SL[6] | 0,2 |
| Natriumsulfit | 0,5 |
| Natriumascorbat | 0,5 |
| konz. Ammoniak | auf pH 10 |
| Wasser | ad 100 |

[1] Texapon(R)N 25 : Fettalkohol $C_{12}$-$C_{14}$ + 2 Ethylenoxid-sulfat, Natriumsalz (28 Gew.-% in Wasser) (Fa. Henkel)

[2] Dehyton(R)K: Fettsäureamid-Derivat aus Kokosöl mit

Betainstruktur der Formel $R\text{-}CONH\text{-}(CH_2)_3\text{-}N^+(CH_3)_2\text{-}CH_2\text{-}COO^-$ (ca. 30 Gew.-% in Wasser) (Fa. Henkel)

3 Polymer JR(R)400: Kationisches Cellulosederivat (Fa. Union-Carbide)

4 Amin (C): Verbindung C2

5 Amin (D): Kokosalkylamin + 2 Ethylenoxid zugänglich durch Anlagerung von 2 Mol Ethylenoxid an Kokosalkylamin z.B. nach DE-OS 552.268

6 Turpinal(R)SL: Hydroxyethan-1,1-diphosphonsäure (Fa. Henkel)

Zur Herstellung der Oxidationshaarfärbemittelzubereitung wurden zunächst Texapon(R)N 25, Dehyton(R)K, Ölsäure, Propylenglykol sowie gegebenenfalls Amin (C) und/oder Amin (D) gemischt und im Wasserbad auf ca. 80 °C erhitzt (Mischung A). Die Farbstoff-Vorprodukte wurden gemischt und in 10 Gewichtsteilen destilliertem Wasser bei 90 °C unter Zugabe von 0,5-2 Gewichtsteilen konzentrierter Ammoniaklösung gelöst (Mischung B). Die Stabilisatorsalze wurden ebenfalls gemischt und mit 0,2 Gewichtsteilen konzentrierter Ammoniaklösung versetzt. Sodann wurde das Turpinal(R)SL zusammen mit destilliertem Wasser von 90 °C hinzugegeben und die Lösung mit weiterem destillierten Wasser versetzt, bis sie insgesamt 10 Gewichtsteile Wasser enthielt. Es entstand eine klare Lösung der Salze und des Komplexierungsmittels (Mischung C).

Nun wurde zunächst Mischung C und anschließend Mischung B unter Rühren in Mischung A eingebracht. Danach wurden 20 Gewichtsteile einer 5 Gew.-%igen Lösung von Polymer JR(R) 400 (entsprechend 1 Gewichtsteil Aktivsubstanz) unter Rühren hinzugegeben, wobei die Viskosität der Mischung signifikant anstieg. Schließlich wurde mit destilliertem Wasser auf insgesamt 100 Gewichtsteile Mischung aufgefüllt.

Diese Haarfärbemittel Zubereitungen sind niedrig- bis mittelviskose Flüssigkeiten, deren Viskositäten mit einem Viskosimeter Haake-Rotovisko RV 12 bei 20 °C bestimmt wurden.

Die Ergebnisse sind in Tabelle 1 aufgeführt.

Tabelle 1

| | Rezeptur 1 | Rezeptur 2 | Rezeptur 3 |
|---|---|---|---|
| Gewichtsteile Amin (C) | - | 5,4 | 5,4 |
| Gewichtsteile Amin (D) | 2,0 | - | 2,0 |
| Viskosität (mPas) | 700 | 2000 | 11100 |

Entsprechende Formulierungen, die weder Amin (C) noch Amin (D) enthielten, waren nicht stabil.

Die Ergebnisse zeigen klar den stabilisierenden Effekt, der durch den Einsatz der erfindungsgemäßen Amine (C) als Folge des Viskositätsaufbaus auftritt.

Des weiteren wird der synergistische Effekt bei Kombination dieser Amine mit Aminen vom Typ (D) deutlich.

Nach Vermischen je eines Gewichtsteiles der Rezepturen 1 bis 3 mit einem Gewichtsteil 6 gewichtsprozentiger Wasserstoffperoxidlösung zeigten nur die Rezepturen 2 und 3 eine voll zufriedenstellende Haftung auf dem Haar.

## Patentansprüche

**1.** Flüssige Zubereitung für Oxidationshaarfärbemittel, bestehend aus Haarfarbstoff-Vorprodukten und einem wäßrigen oder wäßrig-alkoholischen Träger, enthaltend Seifen (A) und wasserlösliche kationische Polymere (B), dadurch gekennzeichnet,
daß sie zur Stabilisierung Amine der Struktur (C)

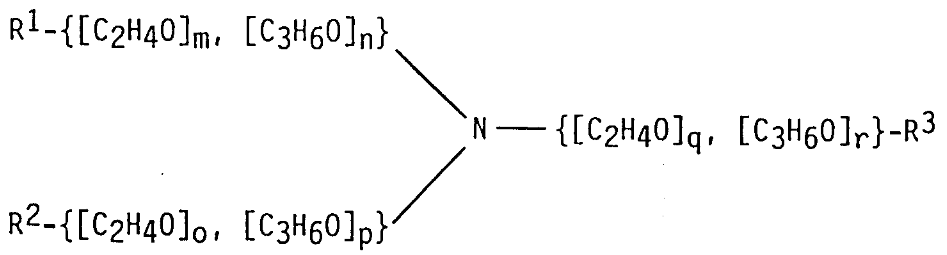

(C),

in denen

| | |
|---|---|
| m, n, o, p, q und r | 0 oder Zahlen bis 6 sind, |
| $R^1$ | eine Alkyl-Gruppe mit 8 bis 22 C-Atomen ist, |
| $R^2$ | Wasserstoff oder eine Alkyl-Gruppe mit 8 bis 22 C-Atomen ist, |
| $R^3$ | Wasserstoff und, falls r = q = 0 ist, eine Alkylgruppe mit 1 bis 4 Kohlenstoffatomen darstellt und im Falle m = n = 0 $R^2$ und $R^3$ nicht gleichzeitig für Wasserstoff stehen |

enthält.

**2.** Flüssige Zubereitung nach Anspruch 1, dadurch gekennzeichnet, daß zur Stabilisierung zusätzlich Amine der Struktur (D)

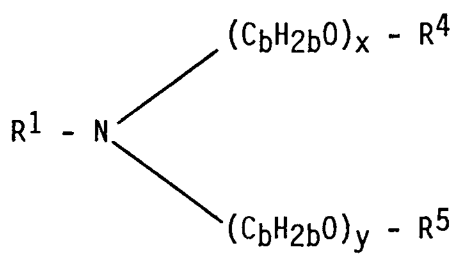

(D)

enthalten sind, in der $R^4$ und $R^5$ unabhängig voneinander Wasserstoff oder eine Acylgruppe der Formel $R^6$-COO- bedeutet, wobei $R^6$ eine Alkyl- oder Alkenylgruppe mit 1-21 C-Atomen ist, b = 2 oder 3 und x und y gleich 0 - 5 sind, wobei die Summe (x + y) 2 bis 6 betragen kann.

**3.** Flüssige Zubereitung nach einem der Ansprüche 1 oder 2, dadurch gekennzeichnet, daß als Seifen (A) Fettsäuren mit 12-22 C-Atomen in Form einer wasserlöslichen Seife, insbesondere Ölsäure in Form von Ammonium- sowie Mono-, Di- oder Triethanolammoniumoleat und als wasserlösliches kationisches Polymeres (B) eine polymere quartäre Ammoniumverbindung aus der Gruppe der

(B1) wasserlöslichen Homo- und Mischpolymerisate mit Einheiten der allgemeinen Formel

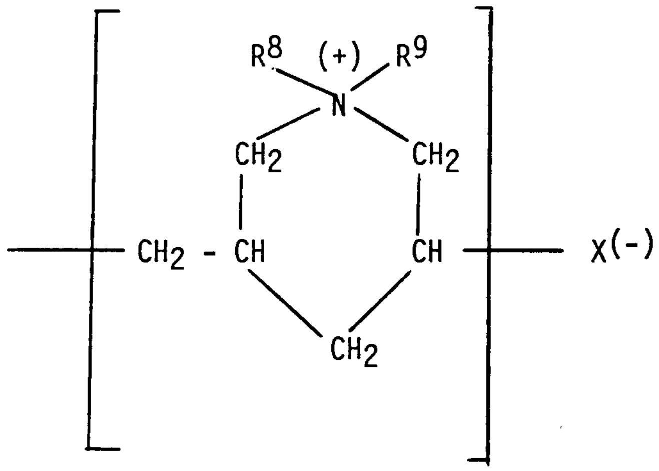

in der $R^8$ und $R^9$ Alkylgruppen mit 1-4 C-Atomen oder Hydroxyalkylgruppen mit 2-4 C-Atomen sind und $X^-$ ein Chlorid-, Bromid-, Hydrogensulfat-, Methoxysulfat-, Phosphat- oder Acetat-Anion ist,
(B2) Celluloseether, deren Anhydroglucoseeinheiten 1-3 über Ethersauerstoff gebundene Substituenten mit quartären Ammoniumgruppen tragen,
(B3) polymeren quartären Harnstoffderivaten mit Einheiten der allgemeinen Formel

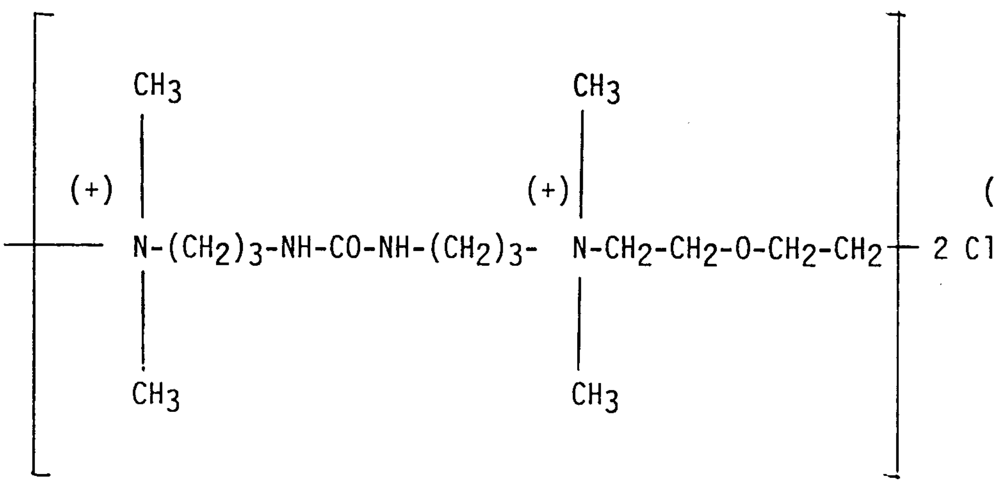

**4.** Flüssige Zubereitung nach einem der Ansprüche 1 bis 3, dadurch gekennzeichnet, daß
1 - 30 Gew.-% Seifen (A),
0,5 - 10 Gew.-% wasserlösliche kationische Polymere (B),
0,1 - 20 Gew.-% Amine (C) sowie
0 - 20 Gew.-% Amine (D) enthalten sind.

**5.** Flüssige Zubereitung nach einem der Ansprüche 1 bis 4, dadurch gekennzeichnet, daß
1 - 30 Gew.-% Seifen (A),
0,5 - 10 Gew.-% wasserlösliche kationische Polymere (B),
0,5 - 10 Gew.-% Amine (C) sowie
0 - 5 Gew.-% Amine (D) enthalten sind.

**6.** Flüssige Zubereitung nach einem der Ansprüche 1 bis 5, dadurch gekennzeichnet, daß Amine der Struktur (C) enthalten sind, wobei $R^1$ eine Alkyl-Gruppe mit 16 bis 18 C-Atomen, $R^2$ Wasserstoff oder eine Alkyl-Gruppe mit 16 bis 18 C-Atomen und $R^3$ Wasserstoff oder, falls r = q = 0 ist, Methyl darstellt, sowie m, o, r Zahlen zwischen 0 und 3 und n, p, q Zahlen zwischen 0 und 1,5 sind und im

Falle m = n = 0 $R^2$ und $R^3$ nicht gleichzeitig für Wasserstoff stehen.

**7.** Flüssige Zubereitung nach einem der Ansprüche 1 bis 6, dadurch gekennzeichnet, daß niedere Alkohole mit 1-4 C-Atomen und/oder Glycole mit 2-6 C-Atomen in einer Menge von insgesamt 1-30 Gew.-% enthalten sind.

**8.** Flüssige Zubereitung nach einem der Ansprüche 1 bis 7, dadurch gekennzeichnet, daß zusätzlich anionische, zwitterionische und/oder nichtionische Tenside in einer Menge von insgesamt 1 bis 20 Gew.-% enthalten sind.

## Claims

**1.** A liquid preparation for oxidation hair dyes - consisting of hair dye precursors and an aqueous or aqueous-alcoholic carrier - containing soaps (A) and water-soluble cationic polymers (B), characterized in that they contain amines corresponding to formula (C)

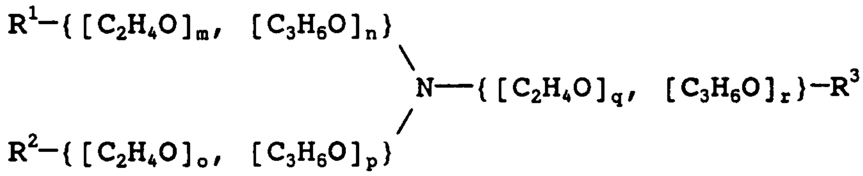
(C)

in which

| | |
|---|---|
| m, n, o, p, q and r | are numbers of 0 to 6, |
| $R^1$ | is a $C_{8-22}$ alkyl group, |
| $R^2$ | is hydrogen or a $C_{8-22}$ alkyl group, |
| $R^3$ | is hydrogen and, where r = q = 0, a $C_{1-4}$ alkyl group and |

where m = n = 0, $R^2$ and $R^3$ cannot both be hydrogen, for stabilization.

**2.** A liquid preparation as claimed in claim 1, characterized in that amines corresponding to formula (D)

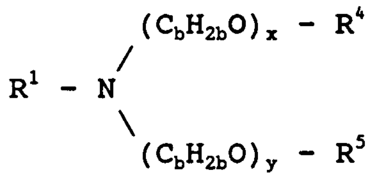
(D)

in which $R^4$ and $R^5$ independently of one another represent hydrogen or an acyl group having the formula $R^6$-COO-, where $R^6$ is a $C_{1-21}$ alkyl or alkenyl group, b = 2 or 3 and x and y are numbers of 0 to 5, the sum (x + y) being from 2 to 6, are additionally present for stabilization.

**3.** A liquid preparation as claimed in claim 1 or 2, characterized in that it contains $C_{12-22}$ fatty acids in the form of a water-soluble soap, more particularly oleic acid in the form of ammonium and mono-, di- or triethanolammonium oleate, as the soaps (A) and a polymeric quaternary ammonium compound from the group consisting of

(B1) water-soluble homopolymers and copolymers containing units corresponding to the following general formula

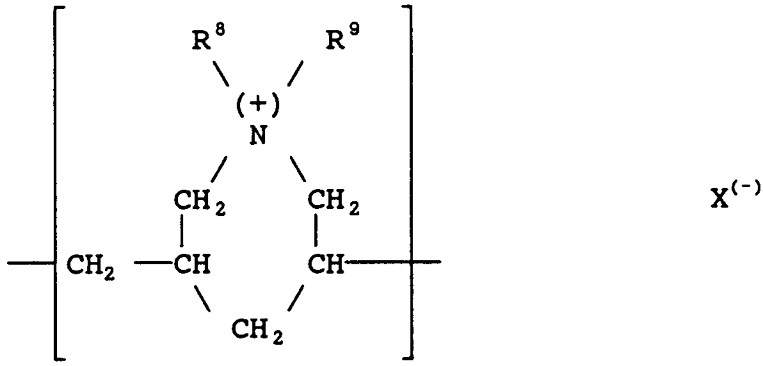

in which $R^8$ and $R^9$ are $C_{1-4}$ alkyl groups or $C_{2-4}$ hydroxyalkyl groups and $X^{(-)}$ is a chloride, bromide, hydrogen sulfate, methoxysulfate, phosphate or acetate ion,
(B2) cellulose ethers of which the anhydroglucose units 1-3 bear substituents containing quaternary ammonium groups which are attached by ether oxygen,
(B3) polymeric quaternary urea derivatives containing units corresponding to the following general formula

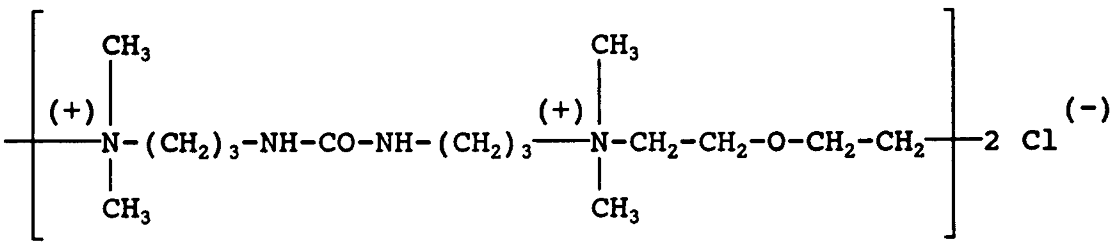

as the water-soluble cationic polymer (B).

**4.** A liquid preparation as claimed in any of claims 1 to 3, characterized in that it contains
1 to 30% by weight soaps (A),
0.5 to 10% by weight water-soluble cationic polymers (B),
0.1 to 20% by weight amines (C) and
0 to 20% by weight amines (D).

**5.** A liquid preparation as claimed in any of claims 1 to 4, characterized in that it contains
1 to 30% by weight soaps (A),
0.5 to 10% by weight water-soluble cationic polymers (B),
0.5 to 10% by weight amines (C) and
0 to 5% by weight amines (D).

**6.** A liquid preparation as claimed in any of claims 1 to 5, characterized in that it contains amines corresponding to formula (C) in which $R^1$ is a $C_{16-18}$ alkyl group, $R^2$ is hydrogen or a $C_{16-18}$ alkyl group and $R^3$ is hydrogen or, where $r = q = 0$, a methyl radical and m, o, r are numbers of 0 to 3 and n, p, q are numbers of 0 to 1.5 and, where $m = n = 0$, $R^2$ and $R^3$ cannot both be hydrogen.

**7.** A liquid preparation as claimed in any of claims 1 to 6, characterized in that it contains lower $C_{1-4}$ alcohols and/or $C_{2-6}$ glycols in a total quantity of 1 to 30% by weight.

**8.** A liquid preparation as claimed in any of claims 1 to 7, characterized in that anionic, zwitterionic and/or nonionic surfactants are additionally present in a total quantity of 1 to 20% by weight.

**Revendications**

1. Préparation liquide pour les teintures d'oxydation pour cheveux, constituée de précurseurs de colorants capillaires et d'un support aqueux ou aqueux-alcoolique, renfermant des savons (A) et des polymères cationiques solubles dans l'eau (B), caractérisée en ce qu'elle contient, pour la stabilisation, des amines de structure (C)

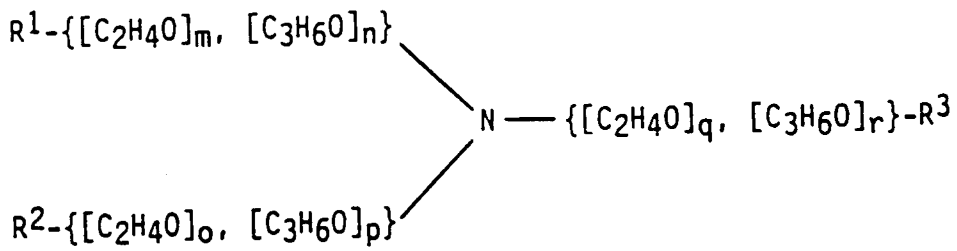

(C),

dans lesquelles m, n, o, p, q et r représentent 0 ou un nombre allant jusqu'à 6,
$R^1$ représente un groupe alkyle comportant 8 à 22 atomes de C,
$R^2$ correspond à l'hydrogène ou à un groupe alkyle possédant 8 à 22 atomes de C,
$R^3$ représente l'hydrogène et, au cas où r = q = 0, un groupe alkyle comportant 1 à 4 atomes de carbone et
dans le cas où m = n = 0, $R^2$ et $R^3$ ne représentent pas simultanément l'hydrogène.

2. Préparation liquide selon la revendication 1, caractérisée en ce qu'elle contient en plus pour la stabilisation, des amines de la structure (D)

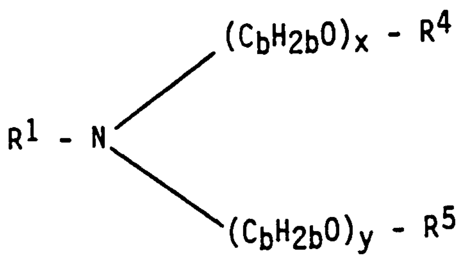

(D)

dans laquelle $R^4$ et $R^5$ représentent indépendamment l'un de l'autre l'hydrogène ou un groupe acyle de formule $R^6$-COO-, dans laquelle $R^6$ correspond à un groupe alkyle ou alcényle comportant 1 à 21 atomes de C, b est égal à 2 ou à 3, tandis que x et y ont une valeur allant de 0 à 5, la somme (x + y) pouvant atteindre 2 à 6.

3. Préparation liquide selon la revendication 1 ou 2, caractérisée en ce qu'elle renferme comme savon (A), des acides gras comportant 12 à 22 atomes de C, sous la forme d'un savon soluble dans l'eau, en particulier l'acide oléique sous la forme d'oléate d'ammonium ainsi que d'oléate de mono-, de di- ou de triéthanolammonium et comme polymère cationique soluble dans l'eau (B), un composé d'ammonium quaternaire polymère faisant partie du groupe des

(B1) homo- et copolymères solubles dans l'eau avec des unités de la formule générale

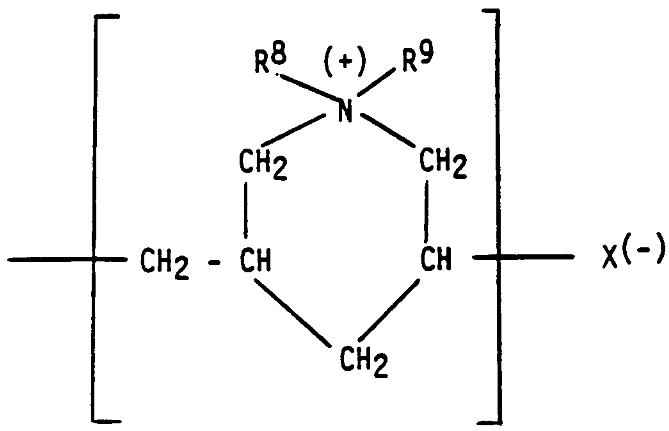

dans laquelle $R^8$ et $R^9$ représentent des groupes alkyle comportant 1 à 4 atomes de C ou des groupes hydroxyalkyle possédant 2 à 4 atomes de C, tandis que $X^{(-)}$ est un anion chlorure, bromure, hydrogénosulfate, méthoxysulfate, phosphate ou acétate,
(B2) éthers cellulosiques, dont les unités d'anhydroglucose portent 1 à 3 substituants liés par de l'oxygène d'éther avec des groupes d'ammonium quaternaires,
(B3) des dérivés d'urée quaternaires polymères avec des unités de la formule générale

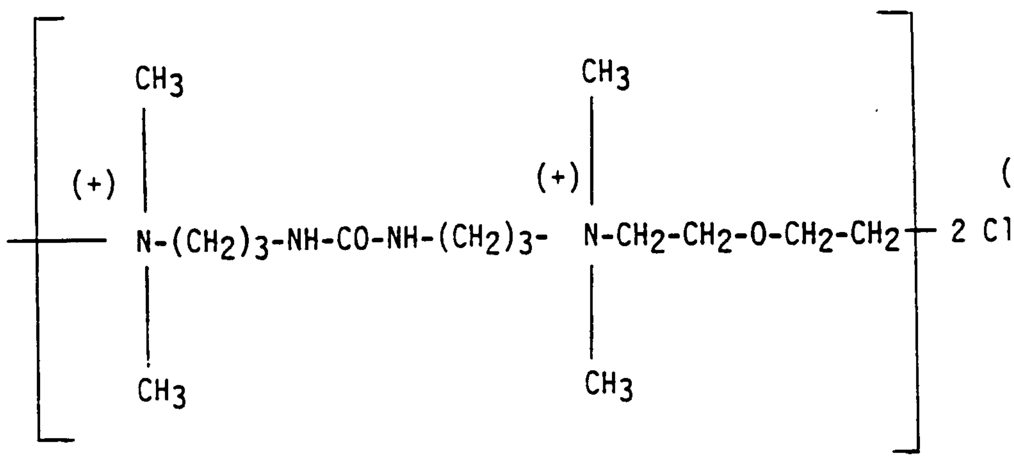

**4.** Préparation liquide selon l'une des revendications 1 à 3, caractérisée en ce qu'elle contient
1 à 30 % en poids de savons (A),
0,5 à 10 % en poids de polymères cationiques solubles dans l'eau (B),
0,1 à 20 % en poids d'amines (C) et
0 à 20 % en poids d'amines (D).

**5.** Préparation liquide selon l'une des revendications 1 à 4, caractérisée en ce qu'elle contient
1 à 30 % en poids de savons (A),
0,5 à 10 % en poids de polymères cationiques solubles dans l'eau (B),
0,5 à 10 % en poids d'amines (C) et
0 à 5 % en poids d'amines (D).

**6.** Préparation liquide selon l'une des revendications 1 à 5, caractérisée en ce qu'elle renferme des amines de la structure (C), dans laquelle $R^1$ représente un groupe alkyle comportant 16 à 18 atomes de C, $R^2$ représente l'hydrogène ou un groupe alkyle comportant 16 à 18 atomes de C et $R^3$ représente l'hydrogène ou, au cas où r = q = 0, un groupe méthyle, tandis que m, o et r correspondent à des chiffres compris entre 0 et 3, et n, p et q, à des chiffres comprises entre 0 et 1,5,

et dans le cas où m = n = 0, $R^2$ et $R^3$ ne peuvent représenter simultanément l'hydrogène.

**7.** Préparation liquide selon l'une des revendications 1 à 6, caractérisée en ce qu'elle contient des alcools inférieurs comportant 1 à 4 atomes de C et/ou des glycols possédant 2 à 6 atomes de C, dans une proportion totale comprise entre 1 et 30 % en poids.

**8.** Préparation liquide selon l'une des revendications 1 à 7, caractérisée en ce qu'elle contient en plus des tensioactifs anioniques, zwitterioniques et/ou non ioniques dans une proportion totale comprise entre 1 et 20 % en poids.